# EUROPEAN PATENT SPECIFICATION

(11) **EP 3 669 154 B1**
(45) Date of publication and mention of the grant of the patent: **19.06.2024**
(21) Application number: 18846047.1
(22) Date of filing: 16.08.2018
(51) Int. Cl.: G01L 9/00, G01L 19/08, G01L 9/14, G01L 19/14, A61B 5/00, A61B 5/03

(54) **MINIATURE IMPLANTABLE WIRELESS PRESSURE SENSOR**
IMPLANTIERBARER DRAHTLOSER MINIATURDRUCKSENSOR
CAPTEUR DE PRESSION SANS FIL IMPLANTABLE MINIATURE

(30) Priority: 16.08.2017 US 201762546455 P
(43) Date of publication of application: 24.06.2020
(73) Proprietor: The Regents of the University of California, Oakland, CA 94607 (US)
(72) Inventor: BACHMAN, Mark, Irvine, CA 92697-2800 (US); BABIKIAN, Sarkis, Irvine, CA 92697-2700 (US); LI, Guann-Pyng, Irvine, CA 92697-2800 (US)
(74) Representative: Puchberger & Partner Patentanwälte
(86) International application number: PCT/US2018/046806
(87) International publication number: WO 2019/036539

(56) References cited:
- US-A- 3 958 558
- US-A- 5 873 835
- US-A- 5 873 835
- US-A1- 2006 117 859
- US-A1- 2006 154 382
- US-A1- 2008 139 959
- US-A1- 2014 371 624
- US-A1- 2016 310 020
- LAZARUS et al.: "Ferrofluid-based Stretchable Magnetic Core Inductors", J. Phys.: Conf. Ser., vol. 660, no. 012007 2015, pages 1-2, XP020293740, Retrieved from the Internet: URL:http://iopscience.iop.org/article/10.1 088/1742-6596/660/1/012007/pdf

## Description

### CROSS REFERENCE

This application claims priority to U.S. Patent Application No.62/546,455, filed on August 16, 2017.

### FIELD OF INVENTION

The present invention is related to a wireless pressure sensor. More particularly, it is related to a miniature implantable wireless pressure sensor. The pressure sensor facilitates the monitoring of physiological pressures in different parts of human body to provide real-time monitoring for applications such as glaucoma, intracranial hypertension and other pressure related indications.

### BACKGROUND OF THE INVENTION

Recent studies in the management of diseases/injuries such as glaucoma and head trauma have revealed the importance of continuous monitoring of physiological pressure such as intraocular pressure (IOP) and intracranial pressure (ICP). Monitoring of these pressures may make treatment more effective and may facilitate prompt intervention when sudden pressure spikes or unforeseen oscillations occur. Thus, implantable, wireless sensors are crucial to facilitate such continuous monitoring of pressure. These sensors should be small to be less traumatic and easy to implant.

Several methods and devices have been reported for continuous measuring of physiological pressure, and especially for IOP. The reported devices can be categorized into active and passive sensors. Active sensors incorporate batteries and integrated circuit that actively transmit pressure information to a wireless reader, and passive sensors are interrogated by a non-contact external reader.

The passive sensors are advantageous due to having rather simple structures and compatible with miniaturization to scales that are suitable for implantation. Moreover, passive devices are generally preferred since battery technology brings additional size and risk of contamination for the implantable device. Examples of implantable passive pressure transducers are known e.g. from US3958558 A and US5873835 A.

Examples of passive devices include passive radio wave resonators that incorporate a capacitive pressure transducer and can be read by near field magnetic coupling. Capacitive-based pressure sensing is quite common. Most passive devices utilize a resonating circuit that changes its resonate frequency when the capacitance changes.

Capacitive membrane devices rely on a large membrane and a small gap between the membrane and an electrically conducting condenser plate. And thus, they tend to be thin in one dimension, but large in cross section area, like a pancake structure.

This kind of structure makes capacitive-based pressure sensors unsuitable for applications such as glaucoma, intracranial hypertension pressure monitoring.

### SUMMARY OF THE INVENTION

The present invention is about a miniature tube shape wireless pressure sensor (100). The sensor (100) comprises a sensor housing which is a miniature tube (110) having a hollow interior (120), a slidable first end (130) and a fixed second end (140); wherein the first end (130) is a pressure sensing interface.

The pressure sensor also comprises an inductor coil (150) patterned on an exterior side of the tube (110) towards the first end (130) of the tube (110), a capacitor module (160) mounted on the exterior side of the tube (110) towards the second end (140) of the tube (110), wherein the capacitor module (160) and the inductor forms an inductance-capacitance L-C resonator with a resonant frequency.

Besides that, the pressure sensor has an electro-magnetic fluid (170) disposed in the hollow interior (120) of the tube (110) towards the first end (130) of the tube, and an inert gas (180) disposed in the hollow interior (120) of the tube (110) towards the second end (140) of the tube (110).

When an outside pressure is applied to the electro-magnetic fluid (170) through the pressure sensing interface (130), the electro-magnetic fluid (170) slides inside the inductor coil (150) and this movement alters an inductance of the inductor coil (150). When the inductance of the inductor coil (150) is altered by the outside pressure, it causes a change in the resonant frequency of the L-C resonator. The change in the resonant frequency indicates a change in the outside pressure.

### BRIEF DESCRIPTION OF THE DRAWINGS

The features and advantages of the present invention will become apparent from a consideration of following detailed description presented in connection with the accompanying drawings in which:
FIG. 1A-1B are schematic drawings of a pressure sensor (10). FIG 1A shows a pressure (10) utilizing a movement of a magnetic material (19) under an influence of an external pressure (12). FIG. 1B shows a pressure sensor (10) utilizing the movement of an inductor (13) under the influence of the external pressure (12).

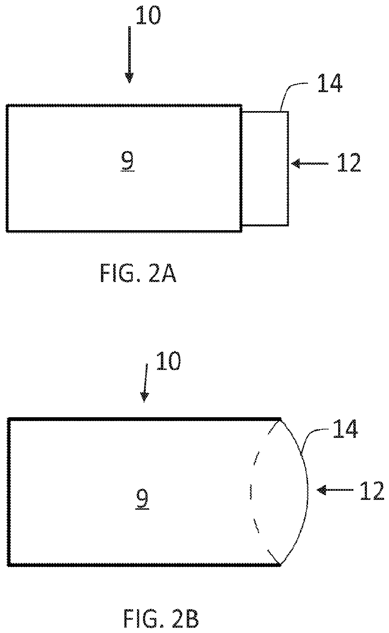

FIG. 2A-2B are schematic drawings showing possible pressure sensor device housing configurations.

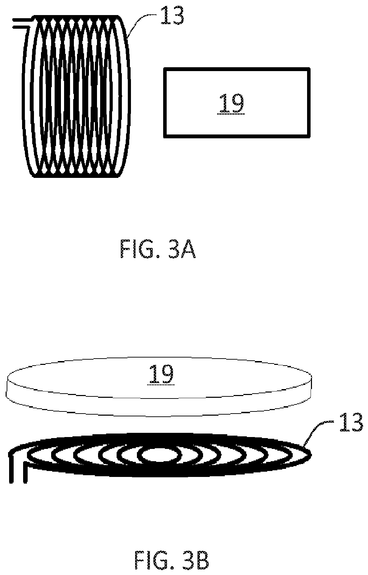

FIG. 3A-3B are schematic drawing showing possible Inductor and magnetic material configurations.
FIG. 4 is a perspective view of an embodiment of the wireless pressure sensor (100).
FIG. 5 is a cross section view of a first embodiment of the wireless pressure sensor (100) shown in FIG. 4.
FIG. 6 is a cross section view of a second embodiment of the wireless pressure sensor (100) shown in FIG. 4.
FIG. 7 is a cross section view of a third embodiment of the wireless pressure sensor (100) shown in FIG. 4.
FIG. 8 illustrates a near field interrogation scheme with magnetic coupling.
FIG. 9 illustrates a backscatter radio wave interrogation scheme.

### DETAIL DESCRIPTION OF THE INVENTION

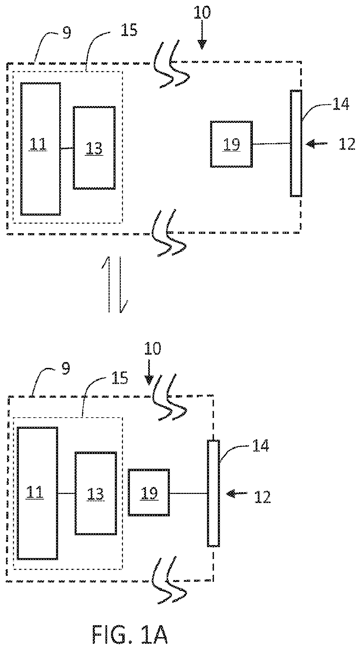

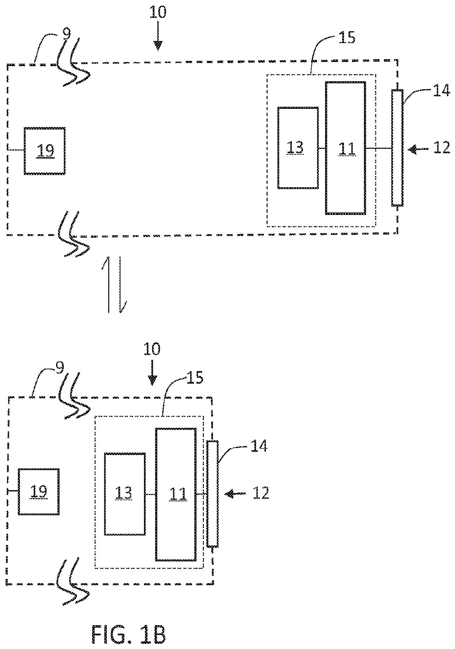

In a broad embodiment, a wireless pressure sensor device (10) comprises a sensor housing (9), a capacitor (11), an inductor (13) and a magnetic material (19). See Figures 1A and 1B,

The capacitor (11) and the inductor (13) are operatively connected to form an inductance-capacitance L-C resonator (15) with a first resonance frequency (17). The magnetic material (19) is at an initial distance from the inductor (13). The sensor housing (9) has a displaceable surface (14) biased to be in an initial resting position (8) by a restorative force (6). In the present invention, the size of the sensor device (10) is about .1 mm to 5 mm long and .1 mm to 5 mm in diameter.

When an external pressure (12) is applied to the displaceable surface (14), the displaceable surface (14) is actuated, thereby creating a shift in the distance between the magnetic material (19) and the inductor (13). The degree of the shift is proportional to the external pressure (12). The shift in distance may be between about .01 mm to 4.9 mm. For example, the shift in distance is about 1 mm.

In some embodiments, if the inductor (13) is stationary, then the magnetic material (19) is moveable such that when the displaceable surface (14) is actuated, the magnetic material (19) moves relative to the inductor (13) to create the shift in distance. As an example, the inductor (13) could remain stationary by being affixed to an interior of the housing (9).

In some embodiments, if the magnetic material (19) is stationary, then the inductor (13) is moveable such that when the displaceable surface (14) is actuated, the inductor (13) moves relative to the magnetic material (19) to create the shift in distance. As an example, the magnetic material (19) could remain stationary by being affixed to an interior of the housing (9).

In some embodiments, the distance shift between the magnetic material (19) and the inductor (13), which is proportional to the external pressure (12), causes a change in the inductance of the inductor (13). Which in turn changes the resonance frequency (17) of the L-C resonator (15). Thereby allowing for detection and measurement of said external pressure (12). The pressure difference detected with this technology could be between 0.5 mmHg to 10 mmHg or about.

The various embodiments provided herein are generally directed to implantable wireless pressure sensors that facilitate the monitoring of physiological pressures in different parts of the human body to provide monitoring for applications such as glaucoma, intracranial hypertension and other pressure related indications. The embodiments are in the form of a small wireless pressure sensor that is implanted in the human body to continuously and directly measure physiological pressures, such as intraocular pressure (IOP), intracranial pressure (ICP) or other pressure. The pressure sensor includes an electric coil and a slidable element that moves when pressure is changed. This movement effectively changes the inductance of the coil. The pressure sensor which has electrical properties of inductance and capacitance acts as L-C resonator. The L-C resonator can be interrogated using external electromagnetic radiation, and thus requires no internal energy source for operation.

The present disclosure provides a wireless pressure sensor utilizing a variable inductive transducer. It comprises an inductor-capacitor circuit also know as L-C resonator tank. The inductor component of the resonator is configured such that its value changes with the ambient pressure. Thus, a change in the ambient pressure cause a change in the inductance value of the circuit, which in turn causes a change in the resonance frequency of the L-C resonator.

Features of the embodiment includes a coil forming an inductor and a movable element that moves in the response to a change in ambient pressure. The movable element is composed of a material that changes the effective inductance of the coil.

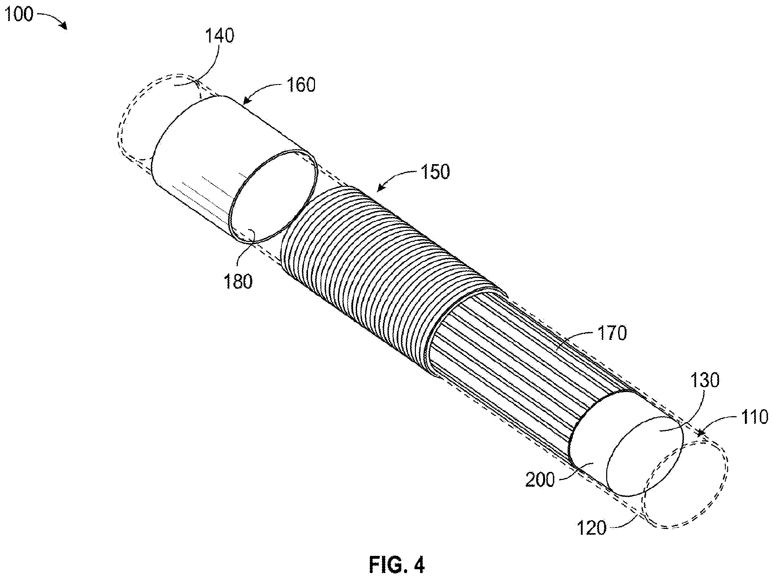

As shown in Figure 4, the exemplary sensor utilizing a variable inductive transducer includes an inductor coil patterned on the wall of a capillary tube. The capillary tube is closed on one end and open on the other end, which serves as the pressure sensing interface or port. The inductor coil is patterned on the opening end of the tube. The capillary tube is filled with an electro-magnetic fluid on the opening end. A capacitor module is mounted towards to the closed end. The inductor and the capacitor form an L-C resonator.

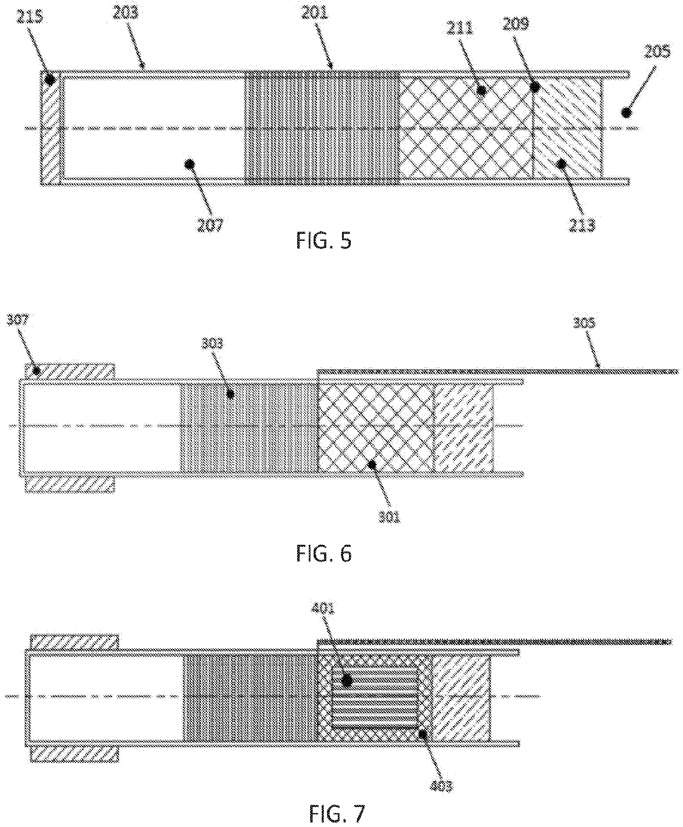

Typical embodiments of the inductive pressure transducer are illustrated in Figures 5, 6 and 7. The first embodiment of the sensor is shown in Figure 5. The sensor consists of an inductor coil (201) patterned or fabricated on the walls of a micro capillary tube or a microchannel (203). The capillary tube (203) is made of a dielectric, chemically inert and flexible material such as fused silica or polymer tubing. The coil (201) may be pattern on either the out walls or the inner walls of the capillary tube (203). The capillary tube (203) is closed on one end and open on the other end (205). The open end serves as a pressure sensing interface or port. The induction coil (201) is patterned towards the open end (205).

The capillary tube (203) is filled with a gas (207) which serves as a pneumatic compression spring. The interface between the inert gas and external environment is comprised of one or two incompressible fluid volumes or regions. A fluid region (209) may have special electrical or magnetic properties that may alter the inductance value of the coil (201) when pushed into the region of the capillary tube (203) overlapped with the coil (201). Fluid (211) can be impregnated with soft magnetic nano-particles such as ferrites (iron oxides) or other variants of alloy particles or nano-engineered particles with magnetic properties. In this example, the fluid 211 is called a ferrofluid or magnetic fluid.

A capacitor module (215) is disposed at the closed end of the capillary tube (203) and forms an L-C resonator with the coil (201). In this embodiment, the coil (201) also functions as an antenna of the sensor.

When the magnetic fluid (211) moves inside the coil (201) under the influence of outside pressure, the relative magnetic permeability of the core of the coil (201) is altered by the fluid (211), which in turn alters the inductance of the coil (201) and the resonance frequency of the L-C resonator. A viscous fluid (213) is used to isolate the capillary fluid (211) from the external environment fluid.

In another embodiment, shown in Figure 6. The fluid (301) may have high electrical conductivity, such as electrolytic fluids, metal salts. The coil (303) is fabricated on the internal walls of the capillary tube. The conductive fluid (301) shunts the turns of the coil (303) as it gets displaced inside the capillary tube and flows over the coil's bare conductor. Thus, the coil (303) is effectively shortened in length. Therefore, the inductance and the L-C resonance frequency is changed.

Also, alternatively, the capacitor (307) of the resonator may be fabricated on the walls of the capillary by deposition of multiple layers of conductive and dielectric materials.

An optional or additional antenna (305) may be added to the device, the geometry of this antenna is defined by the interrogation method discussed below.

In another embodiment as shown in Figure 7, the fluid core may be replaced with a solid material (401) that may have magnetic or electric properties as per discussion above, and that can move in response to a pressure change. In this case, a viscous bearing (403) may be added.

Similar embodiments may be envisioned utilizing a compressible material instead of a gas filled pneumatic cavity to provide the restoring force against the external pressure.

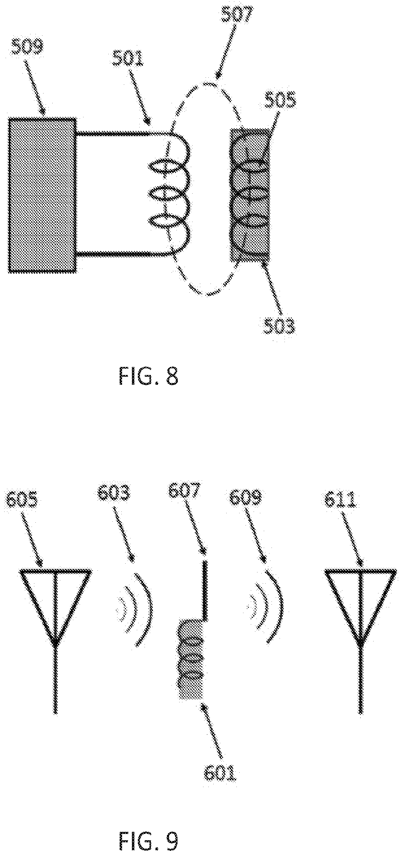

For example, the wireless interrogation of this sensor may be done through two methods utilizing radio frequency signals. Figure 8 describes a first method of using inductive coupling. In this method, the probe antenna (501) is a loop antenna placed at proximity to the sensor (503). The inductor coil (505) of the sensor (503) may serve as the sensor antenna, or an additional loop antenna may be added to the sensor (503). The near field magnetic line (507) of the probe antenna (501) couples with the sensor antenna (505). Thus, an inductive coupling between the probe (501) and sensor (503) is established. A change in pressure is seen as a change in the electrical response at the probe terminals. This may be directly read by the electronic circuitry (509).

In another embodiment, the wireless interrogation can be done using radio frequency signals and the backscattering property of antenna as shown in Figure 9. In this method, the sensor (601) is interrogated with electromagnetic waves. The incident radio wave (603) (coming from the excitation antenna (605)) on a sensor's antenna (607) causes a current to flow through the sensor's antenna (607) and the L-C resonator connected to it. If the radio wave frequency matches with the resonance frequency, a large current would flow in the circuit, which in turn would generate its own electromagnetic radiation. The backscatter wave (609) may be detected by a receiving antenna (611) on the probe side. Thus, the resonance frequency, which is related to the pressure value, of the sensor (601) is determined. The excitation and receiving antenna may be physically one antenna or two separate antennas.

## Claims

1. A wireless pressure sensor device comprising:
a sensor housing (203) closed on one end and open on the other end (205);
a capacitor (215, 307) disposed on the sensor housing (203);
an inductor coil (201, 303) patterned on a wall of the sensor housing (203), wherein the inductor coil (201, 303) and the capacitor (215, 307) are operatively connected to form an inductance-capacitance L-C resonator having a resonant frequency;
a material (211, 301, 401) disposed in the sensor housing (203), the material (211, 301, 401) having conductive and/or magnetic properties;
a gas volume (207) disposed within the sensor housing (203) between a first end of the material (211, 301, 401) and the closed end of the sensor housing (203), the gas volume (207) serving as a compression spring;
a viscous fluid (213) disposed within the sensor housing (203) between the second end of the material (201, 301, 401) and the open end (205) of the sensor housing (203);
wherein when an external pressure is applied to the open end (205) of the sensor housing (203), the external pressure moves the material (211, 301, 401) within the sensor housing (203) with respect to the inductor coil (201, 303), thereby altering the inductance of the inductor coil (201, 303) and changing the resonant frequency of the L-C resonator, thereby allowing for detection and measurement of said external pressure.

2. The pressure sensor of claim 1, wherein the material (211) comprises a ferrofluid (211).

3. The pressure sensor of claim 1, wherein the material (401) comprises a solid material (401) having conductive and/or magnetic properties.

4. The pressure sensor of claim 3, wherein the solid material (401) is surrounded by a viscous bearing (403).

5. The pressure sensor of claim 1, wherein the inductor coil (303) is disposed on an inside surface of the sensor housing (203) and the material (301) comprises a conductive fluid (301), whereby contact between the conductive fluid (301) and the inductor coil (303) shunts turns of the inductor coil (303), thereby changing the resonant frequency of the L-C resonator.

6. The pressure sensor of claim 1, wherein a shape of the sensor housing (203) is a tube.

7. The pressure sensor of claim 1, wherein a size of the sensor is at millimeter scale.

8. The pressure sensor of claim 1, wherein the resonance frequency is measurable wirelessly by magnetic coupling or by backscattered radio wave.

9. The sensor of claim 1, wherein the capacitor (307) comprises multiple layers of conductive and dielectric materials disposed on walls of the sensor housing (203).

10. The sensor of claim 1 further comprising an antenna.

11. The sensor of claim 10, wherein the inductor coil (201) serves as the antenna.

12. The sensor of claim 1 configured to detect and measure a fluid pressure.

## Patentansprüche

1. Drahtlose Drucksensorvorrichtung, umfassend:
ein Sensorgehäuse (203), das an einem Ende geschlossen und am anderen Ende offen ist (205);
einen Kondensator (215, 307), der am Sensorgehäuse (203) angeordnet ist;
eine Induktionsspule (201, 303), die auf einer Wand des Sensorgehäuses (203) strukturiert ist, wobei die Induktionsspule (201, 303) und der Kondensator (215, 307) funktionell verbunden sind, um einen induktiv-kapazitiven L-C-Resonator zu bilden, der eine Resonanzfrequenz aufweist;
ein Material (211, 301, 401), das in dem Sensorgehäuse (203) angeordnet ist, wobei das Material (211, 301, 401) leitende und/oder magnetische Eigenschaften aufweist;
ein Gasvolumen (207), das innerhalb des Sensorgehäuses (203) zwischen einem ersten Ende des Materials (211, 301, 401) und dem geschlossenen Ende des Sensorgehäuses (203) angeordnet ist, wobei das Gasvolumen (207) als eine Druckfeder dient;
ein viskoses Fluid (213), das innerhalb des Sensorgehäuses (203) zwischen dem zweiten Ende des Materials (201, 301, 401) und dem offenen Ende (205) des Sensorgehäuses (203) angeordnet ist;
wobei, wenn ein äußerer Druck auf das offene Ende (205) des Sensorgehäuses (203) ausgeübt wird, der äußere Druck das Material (211, 301, 401) innerhalb des Sensorgehäuses (203) in Bezug auf die Induktionsspule (201, 303) bewegt, wodurch die Induktivität der Induktionsspule (201, 303) geändert wird und die Resonanzfrequenz des L-C-Resonators verändert wird, wodurch die Erfassung und Messung des äußeren Drucks ermöglicht wird.

2. Drucksensor nach Anspruch 1, wobei das Material (211) ein Ferrofluid (211) umfasst.

3. Drucksensor nach Anspruch 1, wobei das Material (401) ein festes Material (401) umfasst, das leitende und/oder magnetische Eigenschaften aufweist.

4. Drucksensor nach Anspruch 3, wobei das feste Material (401) von einem viskosen Lager (403) umgeben ist.

5. Drucksensor nach Anspruch 1, wobei die Induktionsspule (303) auf einer Innenfläche des Sensorgehäuses (203) angeordnet ist und das Material (301) ein leitfähiges Fluid (301) umfasst, wodurch der Kontakt zwischen dem leitfähigen Fluid (301) und der Induktionsspule (303) Windungen der Induktionsspule (303) überbrückt und dadurch die Resonanzfrequenz des L-C-Resonators verändert.

6. Drucksensor nach Anspruch 1, wobei eine Form des Sensorgehäuses (203) ein Rohr ist.

7. Drucksensor nach Anspruch 1, wobei eine Größe des Sensors im Millimeterbereich liegt.

8. Drucksensor nach Anspruch 1, wobei die Resonanzfrequenz durch magnetische Kopplung oder durch rückgestreute Radiowellen drahtlos messbar ist.

9. Sensor nach Anspruch 1, wobei der Kondensator (307) mehrere Schichten aus leitenden und dielektrischen Materialien umfasst, die an Wänden des Sensorgehäuses (203) angeordnet sind.

10. Sensor nach Anspruch 1, der ferner eine Antenne umfasst.

11. Sensor nach Anspruch 10, wobei die Induktionsspule (201) als die Antenne dient.

12. Sensor nach Anspruch 1, der so konfiguriert ist, dass er einen Fluiddruck erfasst und misst.

## Revendications

1. Dispositif de capteur de pression sans fil comprenant:
un boîtier de capteur (203) fermé à une extrémité et ouvert à l'autre extrémité (205);
un condensateur (215, 307) disposé sur le boîtier de capteur (203);
une bobine d'induction (201, 303) disposée sur une paroi du boîtier de capteur (203), la bobine d'induction (201, 303) et le condensateur (215, 307) étant reliés de manière opérationnelle pour former un résonateur L-C d'inductance-capacité ayant une fréquence de résonance;
un matériau (211, 301, 401) disposé dans le boîtier de capteur (203), le matériau (211, 301, 401) ayant des propriétés conductrices et/ou magnétiques;
un volume de gaz (207) disposé dans le boîtier de capteur (203) entre une première extrémité du matériau (211, 301, 401) et l'extrémité fermée du boîtier de capteur (203), le volume de gaz (207) servant de ressort de compression;
un fluide visqueux (213) disposé à l'intérieur du boîtier de capteur (203) entre la deuxième extrémité du matériau (201, 301, 401) et l'extrémité ouverte (205) du boîtier de capteur (203);
dans lequel, lorsqu'une pression externe est appliquée à l'extrémité ouverte (205) du boîtier de capteur (203), la pression externe déplace le matériau (211, 301, 401) à l'intérieur du boîtier de capteur (203) par rapport à la bobine d'induction (201, 303), modifiant ainsi l'inductance de la bobine d'induction (201, 303) et changeant la fréquence de résonance du résonateur L-C, permettant ainsi la détection et la mesure de ladite pression externe.

2. Capteur de pression selon la revendication 1, dans lequel le matériau (211) comprend un ferrofluide (211).

3. Capteur de pression selon la revendication 1, dans lequel le matériau (401) comprend un matériau solide (401) ayant des propriétés conductrices et/ou magnétiques.

4. Capteur de pression selon la revendication 3, dans lequel le matériau solide (401) est entouré d'un palier visqueux (403).

5. Capteur de pression selon la revendication 1, dans lequel la bobine d'induction (303) est disposée sur une surface intérieure du boîtier de capteur (203) et le matériau (301) comprend un fluide conducteur (301), le contact entre le fluide conducteur (301) et la bobine d'induction (303) shuntant les tours de la bobine d'induction (303), modifiant ainsi la fréquence de résonance du résonateur L-C.

6. Capteur de pression selon la revendication 1, dans lequel la forme du boîtier de capteur (203) est un tube.

7. Capteur de pression selon la revendication 1, dans lequel la taille du capteur est à l'échelle du millimètre.

8. Capteur de pression selon la revendication 1, dans lequel la fréquence de résonance est mesurable sans fil par couplage magnétique ou par onde radio rétrodiffusée.

9. Capteur selon la revendication 1, dans lequel le condensateur (307) comprend plusieurs couches de matériaux conducteurs et diélectriques disposées sur les parois du boîtier de capteur (203).

10. Capteur selon la revendication 1, comprenant en outre une antenne.

11. Capteur selon la revendication 10, dans lequel la bobine d'induction (201) sert d'antenne.

12. Capteur selon la revendication 1, configuré pour détecter et mesurer la pression d'un fluide.
